# EUROPEAN PATENT APPLICATION

(11) **EP 4 647 501 A2**
(43) Date of publication of application: **12.11.2025**
(21) Application number: 25205725.2
(22) Date of filing: 31.10.2022
(51) Int. Cl.: C12P 13/04

(54) **NOVEL MICROORGANISM, NOVEL MICROORGANISM CULTURE OR EXTRACT, AND ERGOTHIONEINE PRODUCTION METHOD**

(30) Priority: 05.01.2022 JP 2022000318
(62) Divisional of application: 22918711.7
(71) Applicant: Kureha Corporation, Chuo-ku Tokyo 103-8552 (JP); National Institute of Advanced Industrial Science and Technology, Chiyoda-ku Tokyo 100-8921 (JP)
(72) Inventor: KOSHIYAMA, Tatsuyuki, Tokyo, 103-8552 (JP); HIGASHIYAMA, Yukihiro, Tokyo, 103-8552 (JP); SATO, Shun, Tsukuba-shi, Ibaraki, 305-8560 (JP); MORITA, Tomotake, Tsukuba-shi, Ibaraki, 305-8560 (JP); SAIKA, Azusa, Tsukuba-shi, Ibaraki, 305-8560 (JP); USHIMARU, Kazunori, Tsukuba-shi, Ibaraki, 305-8560 (JP)
(74) Representative: Hoefer & Partner Patentanwälte mbB

(57) **Abstract**

The present disclosure relates to a microorganism (NITE BP-03572) belonging to Rhodosporidiobolus azoricus or to a microorganism (NITE BP-03573) belonging to the genus Vanrija.

## Description

### TECHNICAL FIELD

The present invention relates to a novel microorganism, a novel microorganism culture or extract, and a method for producing ergothioneine.

### BACKGROUND ART

Ergothioneine is one of sulfur-containing amino acids. Ergothioneine has a higher antioxidant effect than that of vitamin E, and has attracted attention as a highly useful compound in the fields of health, beauty and the like.

For example, Patent Document 1 and Non-Patent Document 1 describe transformed filamentous fungi with enhanced ergothioneine production capability.

Non-Patent Document 2 describes a transformed microorganism of the genus Methylobacrium with enhanced ergothioneine production capability. Non-Patent Document 2 describes that microorganisms of the genera Aureobasidium and Rhodotorula have ergothioneine production capability.

Non-Patent Document 3 describes that a microorganism of the genus Pleurotus has ergothioneine production capability.

Patent Document 2 describes that microorganisms of the genera Methylobactrium and Rhodotorula have ergothioneine production capability. Patent Document 3 describes that a microorganism of the genus Moniliella has ergothioneine production capability. Patent Document 4 describes that microorganisms of the genera Dirkmeia, Papiliotrema, and Apiotrichum have ergothioneine production capability.

### CITATION LIST

### PATENT DOCUMENT

Patent Document 1: WO 2016/121285
Patent Document 2: WO 2016/121285
Patent Document 3: WO 2019/004234
Patent Document 4: WO 2021/140693

### NON-PATENT DOCUMENT

Non-Patent Document 1: S. Takusagawa, Biosci. Biotechnol. Biochem., 83, 181-184 (2019)
Non-Patent Document 2: Y. Fujitani et al., J. Biosci. Bioeng., 126, 715-722 (2018)
Non-Patent Document 3: SY. Lin, Int. J. Med. Mushrooms, 17, 749-761 (2015)

### SUMMARY OF INVENTION

### TECHNICAL PROBLEM

It is known that ergothioneine is not biosynthesized in the human body, but biosynthesized in some microorganisms. Thus, research and development on microorganisms that produce ergothioneine and modification of microorganisms to enhance the ergothioneine production are in progress, as described in the prior art documents.

Gene recombination techniques can be used to modify microorganisms to enhance the ergothioneine production. However, the ergothioneine produced by the microorganisms cannot be used in the food industry or the like. Accordingly, there is a strong desire to search for microorganisms with high ergothioneine production, which have not been subjected to gene recombination and are unmodified.

The present invention has been made in light of the above problem, and an object thereof is to provide a novel microorganism with high ergothioneine production.

### SOLUTION TO PROBLEM

As a result of screening, the present inventors have found a novel microorganism that has high ergothioneine production, and have completed the present invention.

A microorganism according to one aspect of the present invention is a microorganism (NITE BP-03572) belonging to Rhodosporidiobolus azoricus or a microorganism (NITE BP-03573) belonging to Vanrija sp. belonging to a species closely related to Vanrija humicola.

Also, a method for producing ergothioneine according to one aspect of the present invention includes culturing a microorganism belonging to the genus Rhodosporidiobolus or the genus Vanrija to obtain a culture containing ergothioneine.

### ADVANTAGEOUS EFFECTS OF INVENTION

According to one aspect of the present invention, a microorganism having high ergothioneine production can be provided.

### DESCRIPTION OF EMBODIMENTS

In the present specification, unless otherwise specified, "A to B" representing a numerical range means "A or more (including A and more than A) and B or less (including B and less than B)".

### [Novel microorganism]

The microorganism according to one aspect of the present invention is a microorganism belonging to the genus Rhodosporidiobolus capable of producing ergothioneine, or a microorganism belonging to the genus Vanrija capable of producing ergothioneine.

The microorganism according to one aspect of the present invention has high ergothioneine production. Ergothioneine is one of sulfur-containing amino acids and has excellent antioxidant effect. In addition, the microorganism according to one aspect of the present invention has not been modified by the gene recombination technique or the like, and thus can also be used in the food industry.

### (1. Rhodosporidiobolus azoricus EB152)

Rhodosporidiobolus azoricus EB152 (hereinafter, sometimes abbreviated as "yeast EB152") is a microorganism originally isolated from a leaf of a plant as an isolation source.

The base sequences of the ribosomal RNA gene 26S rDNA-D1/D2 and ITS regions were determined. Homology search by BLAST was performed across the TechnoSuruga Laboratory microorganism identification system (TechnoSuruga Laboratory, Japan) database DB-FU13.0 and the International Nucleotide Sequence Databases (DDBJ/ENA (EMBL)/GenBank). As a result, EB152 was assigned to Rhodosporidiobolus azoricus. Except for the assimilation of maltose and water-soluble starch as carbon sources and the vitamin requirement, it exhibits physiological and biochemical properties almost similar to those of Rhodosporidiobolus azoricus.

Yeast EB152 was deposited at the **NITE** Patent Microorganisms Depositary (NPMD), National Institute of Technology and Evaluation (hereinafter abbreviated as "NITE") (#122, 2-5-8 Kazusakamatari, Kisarazu-shi, Chiba, Japan) (Date of original deposition: December 15, 2021, Accession No.: NITE BP-03572).

A method for culturing yeast EB152 may be performed in accordance with common culture methods for microorganisms of the genus Rhodosporidiobolus. The culture form is batchwise culture using a liquid medium or fed-batch culture in which a carbon source and/or an organic nitrogen source is continuously added to the culture system, and aeration agitation is desirably performed. As the culture medium, a culture medium containing carbon and nitrogen sources that are assimilable by microorganisms belonging to the genus Rhodosporidiobolus or a required nutrient source such as an inorganic salt may be used. The pH for culture is preferably 3 to 8, the culture temperature is preferably 20°C to 30°C, and the incubation time is preferably 2 to 14 days.

### (2. Vanrija sp. EB891)

Vanrija sp. EB891 (hereinafter, sometimes abbreviated as "EB891") is a microorganism originally isolated from a basidiomycete fruiting body as an isolation source.

The base sequences of the ribosomal RNA gene 26S rDNA-D1/D2 and ITS regions were determined. Homology search by BLAST was performed across the TechnoSuruga Laboratory microorganism identification system (TechnoSuruga Laboratory, Japan) database DB-FU13.0 and the International Nucleotide Sequence Databases (DDBJ/ENA (EMBL)/GenBank). As a result, it was indicated that EB891 is closely related to Vanrija humicola. In addition, it exhibits physiological and biochemical properties almost similar to those of Vanrija humicola except that differences were observed in assimilation of carbon source inulin and water-soluble starch, assimilation of nitrogen source nitrate, vitamin requirement, and viability in 50% D-glucose and 10% NaCL/5% glucose.

Yeast EB891 was deposited at the NITE Patent Microorganisms Depositary (NPMD), National Institute of Technology and Evaluation (hereinafter abbreviated as "NITE") (#122, 2-5-8 Kazusakamatari, Kisarazu-shi, Chiba, Japan) (Date of original deposition: December 15, 2021, Accession No.: NITE BP-03573).

A method for culturing yeast EB891 may be performed in accordance with common culture methods for microorganisms of the genus Vanrija. The culture form is batchwise culture using a liquid medium or fed-batch culture in which a carbon source and/or an organic nitrogen source is continuously added to the culture system, and aeration agitation is desirably performed. As the culture medium, a culture medium containing carbon and nitrogen sources that are assimilable by microorganisms belonging to the genus Vanrija or a required nutrient source such as an inorganic salt may be used. The pH for culture is preferably 3 to 8, the culture temperature is preferably 20°C to 30°C, and the incubation time is preferably 2 to 14 days.

### [Culture]

The culture according to one aspect of the present invention is a culture of yeast EB152 or yeast EB891. The culture according to one aspect of the present invention includes, for example, culture supernatants, culture precipitates, culture medium, cultured microbial cells, and treated products of cultured microbial cells, such as crushed products of cultured microbial cells and lyophilized products of cultured microbial cells. The culture according to one aspect of the present invention contains ergothioneine.

### [Extract]

The extract according to one aspect of the present invention is an extract of yeast EB152 or yeast EB891. As used herein, the term "extract of a microorganism" refers to a product obtained by subjecting a microorganism to extraction treatment and a product obtained by subjecting a culture of a microorganism to extraction treatment. Thus, the extract according to one aspect of the present invention can be obtained, for example, by extraction treatment of yeast EB152 or yeast EB891, or extraction treatment of the culture of yeast EB152 or yeast EB891. The extract according to one aspect of the present invention contains ergothioneine.

Examples of the extraction treatment include hot water extraction; solvent extraction by an organic solvent or the like; pressurized extraction; chemical extraction by an enzyme, a surfactant, or the like; ultrasonic extraction; alkali extraction; acid extraction; extraction by osmotic pressure; extraction by pulverization; extraction by mashing; extraction by freeze-thawing; extraction by liquid nitrogen; and extraction by high-speed agitation. From the viewpoint of exhibiting an excellent plant growth promoting effect, the extraction treatment is preferably hot water extraction. One type of extraction treatment may be performed, or two or more types of extraction treatments may be performed.

The hot water extraction is an extraction process in which an extraction target is brought into contact with or soaked in hot water for a certain time period. The temperature of water used in the hot water extraction is preferably 40°C or higher and more preferably 60°C or higher.

Examples of the extract according to one aspect of the present invention include a hot water extract; a solvent extract by an organic solvent or the like; a pressurized extract; a chemical extract by an enzyme, a surfactant, or the like; an ultrasonic extract; an alkali extract; an acid extract; an extract by osmotic pressure; an extract by pulverization; an extract by mashing; an extract by freeze-thawing; an extract by liquid nitrogen; and an extract by high-speed agitation, of a microorganism namely yeast EB152 or yeast EB891.

Examples of applications of the culture or extract according to one aspect of the present invention include a plant growth regulator containing the culture or extract as an active ingredient.

### [Method for producing ergothioneine]

The method for producing ergothioneine according to one aspect of the present invention includes culturing microorganisms belonging to the genus Rhodosporidiobolus or the genus Vanrija to obtain a culture containing ergothioneine.

In the method for producing ergothioneine according to one aspect of the present invention, the microorganisms belonging to the genus Rhodosporidiobolus are preferably microorganisms belonging to Rhodosporidiobolus azoricus, and more preferably yeast EB152, from the viewpoint of high production of ergothioneine. In addition, from the viewpoint of high production of ergothioneine, the microorganisms belonging to the genus Vanrija are preferably microorganisms belonging to Vanrija sp. belonging to a species closely related to Vanrija humicola, and more preferably yeast EB891.

Collection of ergothioneine from the culture containing ergothioneine may be accomplished, for example, by a common method for collecting and purifying ergothioneine from a microorganism culture. For example, the cultured microbial cells are collected by centrifugation or the like of the culture. Next, the collected microbial cells are subjected to hot water extraction or the like to obtain an extract liquid containing ergothioneine. Ergothioneine can then be collected by purifying the extract liquid. The ergothioneine production of the microorganism can be quantified, for example, by measuring the resulting extract liquid using a high-performance liquid chromatography instrument and a mass spectrometer such as LCMS.

### [Summary]

The microorganism according to a first aspect of the present invention is a microorganism (NITE BP-03572) belonging to Rhodosporidiobolus azoricus or a microorganism (NITE BP-03573) belonging to Vanrija sp. belonging to a species closely related to Vanrija humicola.

The culture according to a second aspect of the present invention is a culture of the microorganism.

The extract according to a third aspect of the present invention is an extract of the microorganism.

The method for producing ergothioneine according to a fourth aspect of the present invention includes culturing the microorganism belonging to the genus Rhodosporidiobolus or the genus Vanrija to obtain a culture containing ergothioneine.

In the method for producing ergothioneine according to a fifth aspect of the present invention, in the fourth aspect described above, the microorganism belonging to the genus Rhodosporidiobolus may be Rhodosporidiobolus azoricus.

In the method for producing ergothioneine according to a sixth aspect of the present invention, in the fourth or fifth aspect described above, the microorganism belonging to the genus Vanrija may be Vanrija sp. belonging to a species closely related to Vanrija humicola.

In the method for producing ergothioneine according to a seventh aspect of the present invention, in any of the fourth to sixth aspects described above, the microorganism belonging to the genus Rhodosporidiobolus may be a microorganism (NITE BP-03572) belonging to Rhodosporidiobolus azoricus.

In the method for producing ergothioneine according to an eighth aspect of the present invention, in any of the fourth to seventh aspects described above, the microorganism belonging to the genus Vanrija may be a microorganism (NITE BP-03573) belonging to Vanrija sp. belonging to a species closely related to Vanrija humicola.

Embodiments of the present invention will be further described in detail using the examples below. The present invention is not limited to the examples below, and it goes without saying that various aspects are possible with regard to the details thereof. Furthermore, the present invention is not limited to the embodiments described above, and it may be varied in various ways within the scope of the claims. Thus, an embodiment achieved by appropriately combining technical means described herein will be included in the technical scope of the present invention. In addition, the contents of all the literatures referred herein are incorporated herein by reference in their entirety.

### EXAMPLES

In the following Examples, the symbol "%" represents % by mass, unless otherwise indicated.

### (1) Enrichment culture using isolation source collected from environment

First, microorganism sampling from environments such as plants and soil was performed twice. As a result, a total of 150 samples (90 samples for the first time and 60 samples for the second time) were collected.

Then, the samples collected were each immersed in a 15-mL plastic tube containing 2 mL of a screening medium, and cultured at 200 rpm and 25°C for 3 to 5 days. The screening medium used was a YM medium containing an antibiotic. Specifically, a medium containing 1% glucose, 0.5% peptone, 0.3% yeast extract, 0.3% malt extract, 0.01% streptomycin sulfate, and 0.005% chloramphenicol was used.

Then, 126 samples (70 samples for the first time and 56 samples for the second time) in which the medium was visually observed to be cloudy (microorganisms proliferated) were selected.

### (2) Selection of samples with oxidative stress load

Culture solutions of the 126 samples selected in (1) above were each diluted 100 or 100000 times in a YM medium. The diluted culture solution was applied to a YM agar medium and a YM agar medium added with 3 mM H₂O₂ (hereinafter abbreviated as H₂O₂-containing YM agar medium), and cultured at 25°C for 2 to 5 days.

The number of colonies having grown on the YM agar medium and the number of colonies having grown on the H₂O₂-containing YM agar medium were counted. Then, 112 samples in which colonies had grown on both the YM agar medium and the H₂O₂-containing YM agar medium were selected.

In addition, for the colonies having grown on the agar medium in the selected 112 samples, the morphology and color were visually observed, and 181 yeast-like colonies of different types (106 colonies for the first time and 75 colonies for the second time) were selected.

### (3) Culture of selected colonies in 96 wells

The 181 colonies selected in (2) above were inoculated into 96 well plates containing 1 mL of a YM medium, and cultured at 1600 rpm and 25°C for 3 to 4 days. After culturing, the collected culture solutions were centrifuged at 2000 rpm and 4°C for 10 minutes. The cell pellets obtained by centrifugation were washed with pure 1 mL and centrifuged again.

To the cell pellets obtained by centrifugation, 0.1 mL of pure water was added to suspend the pellets therein. The resulting suspensions were heated at 96°C for 10 minutes to extract the intracellular components. The extracted intracellular components were then centrifuged to remove microbial cell residues, thereby obtaining extract liquids.

### (4) Quantitative analysis of ergothioneine in extract liquid by LCMS

A mixed solution of 0.15 mL of each of the extract liquids obtained in (3) above and 0.35 mL of acetonitrile was filtered through a 0.45-µm PVDF filter. The resulting filtrate was used as a sample for LCMS measurement.

LCMS-2020, available from Shimadzu Corporation, was used for LCMS analysis. In addition, an Asahipak NH2P-40 2D+ guard column, available from SHODEX, was used as the column for LC. A mixed solution of 10 mM ammonium formate and acetonitrile (10 mM ammonium formate/acetonitrile = 30/70 (v/v)) was used as the mobile phase for LC. The flow rate was set to 0.1 mL/min, and analysis was performed at 25°C.

In MS detection, ionization was performed in DUIS mode for performing ESI ionization and APCI ionization simultaneously. Detection was also performed in SIM mode of m/z = 230 (+) in which ergothioneine could be detected.

As a result of analyzing the extract liquids of the 181 colonies selected in (2) above, 22 colonies with high ergothioneine production (7 colonies for the first time and 15 colonies for the second time) were selected.

### (5) Scale-up culture of ergothioneine-producing microorganism in flask

The 22 colonies selected in (4) above were each inoculated into a 300-mL flask containing 50 mL of a YM medium, and cultured at 200 rpm and 25°C for 7 days (n = 1).

The culture solutions on Days 3 to 7 were collected as appropriate. As in (3) above, after centrifugation and washing of the microbial cells, extract liquids were collected by hot water extraction.

The resulting extract liquids were analyzed by LCMS in a similar manner as in (4) above to select 2 strains (EB152 and EB891) with high ergothioneine production.

Colonies of the selected 2 strains were each inoculated into a 300-mL flask containing 50 mL of a YM medium, and cultured at 200 rpm and 25°C for 5 days (n = 3). The ergothioneine productions on Day 5 of culture were then measured by LCMS.

The ergothioneine productions and production rates of the colonies of the selected 2 strains are shown in Table 1. Table 2 shows the productions and production rates of known microorganisms. In Tables 1 and 2, unless otherwise noted, the unit for the ergothioneine (EGT) production is mg/L, and the unit for the EGT production rate is mg/L/d (ergothioneine production per day). Also, the EGT productions in Table 1 indicate the ergothioneine productions on Day 5 of culture.

As for the "EGT amount in extract" in Table 1, the extract was obtained by aerobically culturing the microorganisms at 25°C for 5 days using a 5-L jar fermenter containing 2 L of a YM medium, and subjecting dried microbial cells after culturing to hot water extraction. Then, the EGT amount in the extract was measured by LCMS.

**[Table 1]**

| Strain name | EGT production (mg/L) | EGT production rate (mg/L/day) | EGT amount in extract (mg/L) |
|---|---|---|---|
| EB152 | 22.6 ± 1.4 | 4.5 | 192 |
| EB891 | 26.3 ± 1.3 | 5.3 | 269 |

**[Table 2]**

| Microorganism | EGT production | EGT production rate | Reference |
|---|---|---|---|
| Aureobasidium pullulans kz25 | 14 | 2 | J Biosci Bioeng 126 (2018) 715 |
| Aspergillus oryzae NSAR1 | 11.5 (mg/kg) | 2.3 (mg/kg/d) | Biosci Biotechnol Biochem 83 (2019) 181 |
| Pleurotus citrinopileatus | 13-98 | 0.8-6.1 | I J Med Mushroom 17 (2015) 749 |
| Methylobacterium aquaticum 22A | 12.2 | 1.7 | J Biosci Bioeng 126 (2018) 715 |

From Tables 1 and 2, it was found that the ergothioneine productions of the selected 2 strains were equal to or higher than the ergothioneine productions of the known ergothioneine-producing microorganisms. It was also found that the ergothioneine production rates of the selected 2 strains were also equal to or higher than the production rates of the known ergothioneine-producing microorganisms. In addition, it was confirmed that the extracts of the selected 2 strains contained ergothioneine abundantly.

### (6) Identification of selected 2 strains

Estimation of the classification groups to which the selected 2 strains were attributed was performed by analysis of the base sequences of the ribosomal RNA gene 26S rDNA-D1/D2 and ITS regions.

### (7) Molecular phylogenetic position and physiological properties of EB152 strain

For the base sequences of the 26S rDNA-D1/D2 regions and ITS-5.8 rDNA in the EB152 strain, homology search by BLAST was performed across the International Nucleotide Sequence Databases. As a result, the base sequences exhibited 98.4 to 100% homology with a plurality of base sequences of Rhodosporidiobolus azoricus as one type of basidiomycetous yeast. In the molecular phylogenetic tree analyzed based on the obtained base sequences, the EB152 strain was included in the phyletic group composed of the genus Rhodosporidiobolus. Then, the same molecular phylogenetic position as that of Rhodosporidiobolus azoricus JCM11251^{T} was indicated.

The EB152 strain was cultured on a YM agar plate medium at 25°C for 3 days, and the colonies formed were observed. The shape of the margin of the colonies was entire, and the raised state thereof was cushion-shaped. The shape of the surface of the colonies was smooth. In addition, the colonies were butter-like, wet, and pink to light orange-colored.

Further, the EB152 strain was cultured on a YM agar plate medium at 25°C for 3 days, and then the cell morphological properties thereof were also observed. It was seen that the nutritive cells were oval to ovoid in shape, and that the strain was proliferated through budding. No formation of sexual reproductive organs was observed in the plate 6 weeks or longer after the start of culture.

The morphological properties of the EB152 strain described above nearly matched the characteristics of Rhodosporidiobolus azoricus to which it was attributed by the DNA sequence analysis of the D1/D2 and ITS regions. The physiological properties of the EB152 strains are shown in Table 3.

In Table 3, the symbol "+" indicates positive. The symbol "-" indicates negative. The letter "W" indicates weakly positive. The letter "D" indicates gradually becoming positive over a period of 1 week or longer after the start of the test, and the letter "L" indicates rapidly becoming positive 2 weeks or longer after the start of the test.

**[Table 3]**

| | | | | | |
|---|---|---|---|---|---|
| <Saccharide fermentation test> | | | | | |
| Glucose | - | | | | |
| <Carbon source assimilation test> | | | | | |
| Glucose | + | Salicin | + | D-mannitol | + |
| Galactose | + | Arbutin | + | Galactitol | + |
| L-sorbose | + | Melibiose | - | Inositol | - |
| D-glucosamine | - | Lactose | - | 2-Keto-D-gluconate | - |
| D-ribose | + | Raffinose | + | 5-Keto-D-gluconate | - |
| D-xylose | + | Melezitose | - | D-gluconate | + |
| L-arabinose | + | Inulin | + | D-glucuronate | - |
| D-arabinose | + | Water soluble starch | W | DL-lactate | - |
| L-rhamnose | - | Glycerol | + | Succinate | W |
| Sucrose | + | Erythritol | - | Citrate | D |
| Maltose | - | Ribitol | + | Methanol | - |
| Trehalose | + | Xylitol | + | Ethanol | + |
| α-Methyl-D- glucoside | | L-arabinitol | D | L-tartaric acid | - |
| Cellobiose | + | D-glucitol | + | | |
| <Nitrogen source assimilation test> | | | | | |
| Nitrate | + | Nitrite | + | | |
| <Resistance test> | | | | | |
| Viability at 25°C | + | Viability at 30°C | + | Viability at 35°C | - |
| 0.1% Cycloheximide | D | 50%(w/v) D-glucose | + | 10% NaCl/5% glucose | W |
| <Vitamin requirement test> | | | | | |
| Vitamin-free medium | + | | | | |

Through the measurements of the molecular phylogenetic position and physiological properties as well as the ergothioneine production, the EB152 strain was determined to be a novel microorganism attributed to Rhodosporidiobolus azoricus.

### (8) Molecular phylogenetic position and physiological properties of EB891 strain

For the base sequences of the 26S rDNA-D1/D2 regions in the EB891 strain, homology search by BLAST was performed across the International Nucleotide Sequence Databases. As a result, the base sequences exhibited 99.0 to 99.8% homology with a plurality of base sequences of Vanrija humicola as one type of basidiomycete yeast. In addition, in the molecular phylogenetic tree analyzed on the basis of the obtained base sequences, it was shown that the EB891 strain was included in a phylogenetic group composed of the genus Vanrija, formed a cluster with Vanrija humicola CBS571^{T}, and was closely related thereto. For the base sequences of ITS-5.8 rDNA in the EB891 strain, homology search by BLAST was performed across the International Nucleotide Sequence Databases. As a result, the base sequences exhibited 98.5 to 100% homology with a plurality of base sequences of Vanrija humicola as one type of basidiomycete yeast. In addition, in the molecular phylogenetic tree analyzed on the basis of the obtained base sequences, it was shown that the EB891 strain was included in a phylogenetic group composed of the genus Vanrija, formed a cluster supported by a high bootstrap value of 99% with a plurality of base sequences of Vanrija humicola, and had a possibility of belonging to Vanrija humicola.

The EB891 strain was cultured on a YM agar plate medium at 25°C for 3 days, and the colonies formed were observed. The shape of the margin of the colonies was entire, and the raised state thereof was flat to cushion-shaped. The shape of the surface of the colonies was smooth to slightly rough. In addition, the colonies were butter-like, wet, and white to cream-colored.

Further, the EB891 strain was cultured on a YM agar plate medium at 25°C for 3 days, and then the cell morphological properties thereof were also observed. It was seen that the nutritive cells were oval to club in shape, and that the strain was proliferated through budding. No formation of sexual reproductive organs was observed in the plate 6 weeks or longer after the start of culture.

The morphological properties of the EB891 strain described above nearly matched the characteristics of Vanrija humicola to which it was attributed by the DNA sequence analysis of the D1/D2 and ITS regions. The physiological properties of the EB891 strain are shown in Table 3.

In Table 4, the symbol "+" indicates positive. The symbol "-" indicates negative. The letter "W" indicates weakly positive. The letter "D" indicates gradually becoming positive over a period of 1 week or longer after the start of the test, and the letter "L" indicates rapidly becoming positive 2 weeks or longer after the start of the test.

**[Table 4]**

| | | | | | |
|---|---|---|---|---|---|
| <Saccharide fermentation test> | | | | | |
| Glucose | - | | | | |
| <Carbon source assimilation test> | | | | | |
| Glucose | + | Cellobiose | + | D-mannitol | + |
| Galactose | + | Salicin | + | Galactitol | + |
| L-sorbose | + | Melibiose | + | Inositol | + |
| D-glucosamine | + | Lactose | + | D-gluconate | + |
| D-ribose | + | Raffinose | D | D-glucuronate | + |
| D-xylose | + | Melezitose | + | DL-lactate | + |
| L-arabinose | + | Inulin | + | Succinate | + |
| D-arabinose | + | Water soluble starch | + | Citrate | + |
| L-rhamnose | + | Glycerol | + | Methanol | - |
| Sucrose | + | Erythritol | + | Ethanol | + |
| Maltose | + | Ribitol | + | Saccharate | + |
| Trehalose | + | Xylitol | + | | |
| α-Methyl-D-glucoside | + | D-glucitol | + | | |
| <Nitrogen source assimil ation test> | | | | | |
| Nitrate | W | | | | |
| <Resistance test> | | | | | |
| Viability at 30°C | + | Viability at 35°C | + | Viability at 37°C | - |
| 0.01% Cycloheximide | + | 50% (w/v) D-glucose | + | 10% NaCl/5% glucose | + |
| <Vitamin requirement test> | | | | | |
| Vitamin-free medium | + | | | | |

Through the measurements of the molecular phylogenetic position and physiological properties as well as the ergothioneine production, the EB891 strain was determined to be a novel microorganism attributed to Vanrija humicola or a species closely related thereto.

As a result of the analysis of the base sequences, it was presumed that the EB152 strain belonged to Rhodosporidiobolus azoricus, and the EB891 strain belonged to Vanrija humicola or a species closely related thereto.

### INDUSTRIAL APPLICABILITY

The microorganisms of the present invention have high ergothioneine production and can be used in the fields of health, beauty, and the like.

### ACCESSION NUMBER

### NITE BP-03572

### NITE BP-03573

The present invention can also be described by means of the following clauses:
[Clause 1] A microorganism (NITE BP-03572) belonging to Rhodosporidiobolus azoricus, or a microorganism (NITE BP-03573) belonging to a species (Vanrija sp.) closely related to Vanrija humicola.
[Clause 2] A culture of the microorganism according to clause 1.
[Clause 3] An extract of the microorganism according to clause 1.
[Clause 4] A method for producing ergothioneine, the method comprising culturing a microorganism belonging to the genus Rhodosporidiobolus or the genus Vanrija to obtain a culture containing ergothioneine.
[Clause 5] The method for producing ergothioneine according to clause 4, wherein the microorganism belonging to the genus Rhodosporidiobolus is Rhodosporidiobolus azoricus.
[Clause 6] The method for producing ergothioneine according to clause 4, wherein the microorganism belonging to the genus Vanrija is a species (Vanrija sp.) closely related to Vanrija humicola.
[Clause 7] The method for producing ergothioneine according to clause 4, wherein the microorganism belonging to the genus Rhodosporidiobolus is a microorganism (NITE BP-03572) belonging to Rhodosporidiobolus azoricus.
[Clause 8] The method for producing ergothioneine according to clause 4, wherein the microorganism belonging to the genus Vanrija is a microorganism (NITE BP-03573) belonging to a species closely related to Vanrija humicola.

## Claims

1. A microorganism NITE BP-03573 belonging to the genus Vanrija.

2. A culture of the microorganism according to claim 1.

3. A method for producing ergothioneine, the method comprising culturing a microorganism belonging to the genus Vanrija to obtain a culture containing ergothioneine.

4. The method for producing ergothioneine according to claim 3, wherein the microorganism belonging to the genus Vanrija is Vanrija sp..

5. The method for producing ergothioneine according to claim 3, wherein the microorganism belonging to the genus Vanrija is a microorganism NITE BP-03573.
